# DEMANDE DE BREVET EUROPEEN

(11) **EP 1 462 085 A1**
(43) Date de publication de la demande: **29.09.2004**
(21) Numéro de dépôt: 04290609.9
(22) Date de dépôt: 05.03.2004
(51) Int. Cl.: A61K 7/043, A45D 31/00

(54) **Composition de maquillage des ongles à effet miroir**

(30) Priorité: 11.03.2003 FR 0303003
(71) Demandeur: L'OREAL, 75008 Paris (FR)
(72) Inventeur: Ramin, Roland, 75014 Paris (FR); Cavazzuti, Roberto, 75015 Paris (FR)
(74) Mandataire: Le Coupanec, Pascale

(57) **Abrégé**

La présente invention concerne une composition pour le maquillage des ongles, caractérisée par le fait qu'elle comprend, dans un milieu physiologiquement acceptable, des particules à reflet métallique en une proportion supérieure ou égale à 2 % en poids, par rapport au poids total de la composition, et au moins un agent texturant ou un mélange d'agents texturants en une proportion inférieure ou égale à 15 % en poids, par rapport au poids total de la composition.

## Description

La présente invention se rapporte à une composition pour le maquillage des ongles utilisant des particules à reflet métallique permettant l'obtention d'un effet miroir. Cette invention se rapporte également à un kit pour le maquillage des ongles en vue de l'obtention d'un maquillage à effet miroir. La présente invention se rapporte en outre aux procédés de maquillage correspondants.

L'utilisation de particules métalliques a déjà été décrite dans différents types de compositions cosmétiques de maquillage. Ainsi, la demande de brevet EP 1 082 952 décrit des compositions de maquillage, notamment des ongles, contenant des particules de verre recouvertes d'une couche métallique permettant l'obtention d'un maquillage présentant un aspect métallique éclatant et résistant à l'usure. Il est également décrit dans la demande de brevet EP 953 330, l'association de deux compositions différentes comprenant respectivement des particules métalliques de type pigment goniochromatique et un pigment de type classique ayant une des couleurs du premier pigment pour l'obtention d'un maquillage à effet métallique variable selon l'angle d'observation et présentant des effets irisés.

Plus récemment, la demande de brevet internationale WO 02/03913 décrit des compositions de vernis à ongles contenant des particules sous forme de plaquettes d'aluminium dans des proportions pondérales de 0,4 à 0,75 % et des agents filmogènes ayant des poids moléculaires élevés pour l'obtention d'un maquillage de type miroir, c'est-à-dire en l'occurrence un maquillage ayant non seulement la couleur de l'aluminium mais également une brillance et une capacité à réfléchir les éléments distincts d'un objet.

Les inventeurs ont découvert qu'il était possible d'obtenir des maquillages présentant un effet miroir amélioré par rapport à ceux obtenus dans l'art antérieur en utilisant des compositions liquides, de faible viscosité, à teneur réduite en agents texturants et à teneur élevée en particules à reflet métallique.

La présente invention concerne, selon un premier aspect, une composition pour le maquillage des ongles, notamment pour un maquillage à effet miroir, comprenant, dans un milieu physiologiquement acceptable, des particules à reflet métallique en une proportion supérieure ou égale à 2 % en poids, par rapport au poids total de la composition, et au moins un agent texturant ou un mélange d'agents texturants en une proportion inférieure ou égale à 15 % en poids, par rapport au poids total de la composition.

La présente invention concerne, selon un deuxième aspect, une composition pour le maquillage des ongles comprenant, dans un milieu physiologiquement acceptable, des particules à reflet métallique et au moins un agent texturant ou un mélange d'agents texturants en une proportion inférieure ou égale à 15 % par rapport au poids total de la composition, ladite composition étant apte à former un film dont la résistance à l'usure exprimée par la perte en masse, mesurée selon la norme AFNOR NF T30-015, est supérieure à 5 % en poids, en particulier supérieure à 10 % en poids et plus particulièrement supérieure à 15 % en poids.

La présente invention concerne, selon un troisième aspect, l'utilisation d'une composition telle que définie précédemment pour former une couche de base dans un maquillage multicouche.

La présente invention concerne, selon un quatrième aspect, un kit pour le maquillage des ongles comprenant, dans des conditionnements séparés, une première composition comprenant, dans un premier milieu physiologiquement acceptable, des particules à reflet métallique en une proportion supérieure ou égale à 2 % en poids, par rapport au poids total de la première composition et au moins un agent texturant ou un mélange d'agents texturants en une proportion inférieure ou égale à 15 % par rapport au poids total de la composition, et une seconde composition, différente de la première composition, comprenant, dans un second milieu physiologiquement acceptable, au moins 10 % en poids par rapport au poids total de la seconde composition d'au moins un agent filmogène.

La présente invention concerne selon un cinquième aspect, un kit pour le maquillage des ongles comprenant dans des conditionnements séparés, une première composition comprenant, dans un premier milieu physiologiquement acceptable, des particules à reflet métallique, ladite première composition étant apte à former un film dont la résistance à l'usure exprimée en perte de masse, mesurée selon la norme AFNOR NF T30-015, est supérieure à 5 % en poids, en particulier à 10 % et plus particulièrement supérieure à 15 % en poids, et une seconde composition, différente de la première composition, comprenant dans un second milieu physiologiquement acceptable, au moins 10 % en poids par rapport au poids de la seconde composition d'au moins un agent filmogène

La présente invention concerne, selon un sixième aspect, un procédé pour le maquillage des ongles comprenant l'application sur tout ou partie de la surface à maquiller d'au moins une couche d'une composition pour le maquillage des ongles telle que définie précédemment.

La présente invention concerne, selon un septième aspect, un procédé de maquillage des ongles comprenant l'application sur la surface à maquiller d'au moins une couche de chacune des compositions d'un kit tel que défini précédemment.

La présente invention concerne, selon un huitième aspect, un support synthétique maquillé comprenant un maquillage susceptible d'être obtenu par le procédé de l'invention.

### Effet miroir

Comme préciser précédemment, l'effet « miroir » se distingue notamment d'un simple effet métallique par sa capacité à réfléchir au moins en partie les éléments distincts d'un objet.

Plus précisément, au sens de l'invention, par « maquillage à effet miroir », on désigne un maquillage qui présente une réflexion spéculaire intense.

En l'occurrence, les films obtenus avec les compositions selon l'invention sont particulièrement avantageux en terme de réflexion lumineuse. Cette réflexion lumineuse peut, le cas échéant, être appréciée selon le test suivant :

Sur une plaque de verre, on dépose une couche de 150 µm d'épaisseur de la composition à tester (avant séchage), puis on laisse sécher pendant vingt-quatre heures à température ambiante. On détermine alors les propriétés de réflexion de la lumière du film obtenu.

En particulier, on peut mesurer à l'aide d'un spectrocolorimètre le pourcentage de réflectance. Par exemple, pour un miroir parfait (procédé du verre argenté), la valeur est proche de 100 %. Pour une formulation traditionnelle de vernis à ongles à effet métallique, la valeur maximale est de 45%. Pour l'effet « miroir » recherché, la valeur est au moins égale à 50%.

En l'occurrence, les compositions selon l'invention sont aptes à former un film possédant un pourcentage de réflectance au moins égal à 50 %, en particulier supérieur ou égal à 70 %.

Les compositions selon l'invention sont généralement fluides, c'est-à-dire de faible viscosité.

Par l'expression «compositions liquides de faible viscosité», on désigne des compositions présentant généralement une viscosité dynamique, à 25 °C et à pression atmosphérique, de l'ordre de 30 à 250 Pa.s en particulier de 50 à 150 Pa.s, mesurée à l'aide d'un viscosimètre Brookfield type LV II.

Vraisemblablement, le fait de formuler les particules à reflet métallique dans une composition de faible viscosité est favorable à l'organisation physique de ces particules au moment de leur application sur le support de maquillage, et en particulier privilégie l'obtention d'une répartition homogène et continue desdites particules conduisant à un effet miroir amélioré.

### Résistance à l'usure

Par ailleurs, lorsque la composition de l'invention est appliquée sur un support, il est possible d'obtenir, après évaporation des composés volatils, une couche de base formée par un film, et notamment un film de faible épaisseur. Il est ainsi possible d'obtenir un film ayant une épaisseur allant de 0,5 à 30 µm.

Le film ainsi obtenu présente une faible résistance à l'usure.

La résistance du film pouvant être obtenu avec les compositions selon l'invention, peut être notamment mesurée selon la norme AFNOR NF T30-015, dont le principe est rappelé ci-après.

On applique la composition à tester sous forme d'une couche de 600 µm d'épaisseur (avant séchage) sur un disque, puis on laisse sécher pendant une heure à 30 °C. Le film de vernis déposé sur le disque est ensuite mis pendant une heure en contact avec des disques abrasifs (abrasimètre TABER), le disque ayant une vitesse de rotation d'un tour par seconde. Au bout d'une heure, on pèse le disque et on calcule la perte de masse PM de produit exprimée en pourcentage du poids perdu par rapport au poids initial.

Par conséquent, dans ce test, plus la perte de poids est élevée, plus le pourcentage de poids perdu est élevé, et plus la résistance à l'usure de la composition est faible.

Le film obtenu avec la composition de l'invention présente une résistance à l'usure exprimée en perte de masse mesurée selon la norme AFNOR NF T 30-015 généralement supérieure à 5 %, en particulier supérieure à 10 % et plus particulièrement supérieure à 15 % en poids.

Selon un mode de réalisation particulier, la composition de l'invention apte à former un film présentant une telle résistance à l'usure, comprend, dans un milieu physiologiquement acceptable, des particules à reflets métalliques en une proportion supérieure ou égale à 2 % en poids par rapport au poids total de la composition.

Cette composition peut comprendre au moins un agent texturant ou un mélange d'agents texturants en une proportion inférieure ou égale à 15 % en poids par rapport au poids total de la composition.

### Les agents texturants

Par « agent texturant », on entend désigner dans le cadre de la présente invention, tout composé organique agissant à titre principal ou secondaire au niveau de la rhéologie de la composition selon l'invention.

En l'occurrence, il peut s'agir d'agents épaississants conventionnels tels que des charges comme les argiles, les silices pyrogénées, les huiles de ricin hydrogénées, les polyamides, les dérivés cellulosiques et/ou d'agents filmogènes comme les dérivés cellulosiques sous forme de polymères cellulosiques (nitrocelluloses, acétobutyrates de cellulose, esters de cellulose et éthers de cellulose) et/ou de résines et/ou d'agents auxiliaires de filmification.

Les agents filmogènes sont principalement destinés à faciliter l'application de la composition et à assurer la formation d'un film tout en permettant néanmoins l'obtention d'une composition liquide de faible viscosité.

La composition de la présente invention est en particulier caractérisée par sa faible proportion en agents texturants. La proportion totale en agents texturants est généralement inférieure ou égale à 15 % en poids, et en particulier inférieure ou égale à 10 % en poids par rapport au poids total de la composition.

Cette proportion est généralement supérieure ou égale à 2 % en poids, et en particulier à 5 % en poids par rapport au poids total de la composition.

Les agents texturants utilisés dans les compositions selon l'invention sont en particulier choisis parmi les agents filmogènes, les résines, les agents auxiliaires de filmification, les agents épaississants et leurs mélanges.

### Les agents filmogènes

Les agents filmogènes comprennent en particulier des polymères filmogènes.

Par « polymère filmogène », on désigne un polymère apte à former à lui seul ou en présence d'un agent auxiliaire de filmification, un film isolable, notamment continu et adhérant sur un support, notamment sur les matières kératiniques.

Dans la composition, on peut utiliser un seul polymère filmogène ou un mélange de polymères filmogènes. Ce polymère filmogène peut être choisi dans le groupe constitué par les polymères radicalaires, les polycondensats et les polymères d'origine naturelle.

Le polymère filmogène peut être organique ou inorganique.

Selon une première variante de l'invention, le polymère filmogène organique est au moins un polymère choisi parmi le groupe comprenant : les polymères filmogènes solubles ou dispersibles dans au moins une classe de solvant organique tel que par exemple les cétones, les alcools, les glycols et éthers de propylène glycols, les esters à chaînes courtes, les alcanes et leurs mélanges.

Les polymères correspondants peuvent être de toute nature chimique. En particulier, ils peuvent résulter soit de l'homo- ou co-polymérisation de monomères insaturés, soit de polycondensation, soit de la modification de polymères naturels, en particulier des polysaccharides. Les masses moléculaires moyennes en poids (Mp) de ces polymères peuvent varier de 3 000 à 1 000 000, notamment de 5 000 à 800 000, et en particulier de 10 000 à 500 000.

Parmi les polymères solubles ou dispersibles dans les solvants organiques, les polymères suivants conviennent tout particulièrement :
a) les homo- et co-polymères esters et/ou amides d'acides (méth)acryliques, en particulier les polymères résultant de la polymérisation ou copolymérisation des acrylates et/ou méthacrylates de méthyle, éthyle, propyle, butyle, isobutyle, tertiobutyle, pentyle, hexyle, cyclohexyle, éthyl 2-hexyle, heptyle, octyle, isobornyle, norbornyle, adamantyle, ou les (méth)acrylamides correspondants. Ces polymères comporteront de préférence de 0 à 20 % d'un comonomère polaire tel que acide (méth)acrylique, (méth)acrylamide, (méth)acrylate d'hydroxyéthyle, (méth)acrylate de 2-hydroxypropyle, et le (méth)acrylonitrile. Ils peuvent également résulter de la copolymérisation avec le styrène ou un styrène substitué.
b) Les homo- et co-polymères esters ou amides vinyliques, en particulier les homo- et co-polymères résultant de la polymérisation de l'acétate de vinyle, propionate de vinyle, versatate de vinyle, avec ou sans présence d'un comonomère polaire tel que les acides crotonique, acide allyloxyacétique, anhydride (ou acide) maléique, anhydride (ou acide) itaconique, vinyl acétamide et vinyl formamide. De même, ils peuvent résulter de la copolymérisation d'au moins un des monomères cités avec du styrène ou un styrène substitué.
c) Les celluloses et dérivés cellulosiques comme les nitrocelluloses et/ou les esters de cellulose tels que les acétates de cellulose, les propionates de cellulose, les butyrates de cellulose, les acétopropionates de cellulose et les acétobutyrates de cellulose.
d) Les polycondensats solubles ou dispersibles dans ces solvants. Ils sont généralement utilisés comme filmogène principal ou bien comme cofilmogène d'une des classes de polymères cités précédemment (a à c), en particulier s'ils sont de faible poids moléculaire (Mp < 20 000). Ils peuvent être choisis parmi les polymères ou copolymères suivants : les polyuréthanes, les polyuréthanes acryliques, les polyurées, les polyuréthanes de polyurée, les polyuréthanes de polyester, les polyuréthanes de polyéther, les polyesters, les polyester-amides, les polyesters à chaîne grasse, les époxys, et les condensats arylsulfonamide et en particulier tosylamide/formaldéhydes,

Parmi ces polycondensats, en particulier si on les utilise comme filmogène ou cofilmogène d'une ou plusieurs nitrocelluloses et/ou d'un ester de cellulose (classe c), on peut plus particulièrement citer :
- les polyesters, en particulier les polyesters à chaîne grasse et plus particulièrement les copolymères de nom CTFA : « copolymère d'anhydride phtalique/glycérol/décanoate de glycidyle » et « copolymère d'acide adipique/néopentylglycol/anhydride trimellitique »
- les alkydes,
- les condensats tosyl amide/formaldéhyde,
- les polyuréthanes et polyurée-uréthanes
- les résines acryliques.
- les résines de silicone (non volatiles ou partiellement volatiles)

Selon une seconde variante de l'invention, le, ou au moins un polymère filmogène peut être choisi parmi les dispersions aqueuses de particules de polymères ou encore latex filmogènes, et dans ce cas la composition selon l'invention comprend au moins une phase aqueuse.

La dispersion aqueuse comprenant un ou plusieurs polymères filmogènes peut être préparée par l'homme du métier sur la base de ses connaissances générales, notamment par polymérisation en émulsion ou par mise en dispersion du polymère préalablement formé.

Parmi les polymères filmogènes de ce type utilisables dans la composition selon la présente invention, on peut citer les polymères synthétiques, de type polycondensat ou de type radicalaire, les polymères d'origine naturelle, et leurs mélanges.

On peut notamment utiliser, mais sous forme de latex, les polymères (homo- et co-polymères) qui sont cités précédemment comme polymères solubles ou dispersibles en milieu solvant organique, et plus particulièrement les polymères des classes a, b et c.

On peut ainsi citer, parmi les polycondensats, les polyuréthanes anioniques, cationiques, non ioniques ou amphotères, les polyuréthanes-acryliques, les polyuréthanes-polyvinylpyrrolidones, les polyester-polyuréthanes, les polyéther-polyuréthanes, les polyurées, les polyurée polyuréthanes, et leurs mélanges.

On peut également citer les polyesters, les polyesters amides, les polyesters à chaîne grasse, les polyamides et les résines époxyesters.

Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.

Les polymères de type radicalaires peuvent être notamment des polymères, ou des copolymères, acryliques et/ou vinyliques. On utilise de préférence des polymères radicalaires anioniques. Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.

Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.

On peut également utiliser des copolymères acryliques/silicones ou encore des copolymères nitrocellulose/acryliques.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

La dispersion peut également comprendre un polymère associatif de type polyuréthane ou une gomme naturelle, telle que la gomme xanthane.

Comme polymère en dispersion aqueuse, on peut citer les dispersions de polymères acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432®, par la société DOW CHEMICAL. On peut également employer des dispersions aqueuses de polyuréthane, et notamment les polyester-polyuréthanes vendues sous les dénominations « AVALURE UR-405® », « AVALURE UR-410® », « AVALURE UR-425® », « SANCURE 2060® » par la société GOODRICH et les polyéther-polyuréthanes vendus sous les dénominations « SANCURE 878® » par la société GOODRICH, « NEOREZ R-970® » par la société AVECIA.

Selon une troisième variante de l'invention, le polymère filmogène peut être choisi parmi les polymères hydrosolubles ou hydrodispersibles, et dans ce cas la composition selon l'invention comprend au moins une phase aqueuse.

Comme polymères hydrodispersibles, on peut citer les polycondensats hydrodispersibles à fonctions sulfonates tels que les copolyesters constitués de motifs dérivant d'acide isophtalique, du sel de sodium de l'acide sulfoisophtalique, de diéthylène glycol et de 1-4 cyclohexane diméthanol, ceux-ci étant en particulier présents dans des proportions de 89/11/78/22 ou de 82/18/54/46. Ces polycondensats sont commercialisés respectivement sous les dénominations de « AQ 38/ » et de « AQ 55/ » par la société EASTMAN KODAK.

Comme polymères hydrosolubles, on peut citer les copolymères hydrosolubles à fonctions acides carboxyliques (polymères synthétiques) qui sont de préférence choisis parmi :
a) les copolymères acétate de vinyle/acide crotonique polyoxyéthylénés,
b) les copolymères N-octylacrylamide/méthacrylate de méthyle/méthacrylate d'hydroxypropyle/acide acrylique/méthacrylate de tert-butylaminoéthyle,
c) les copolymères alternés méthylvinyléther/anhydride maléique monoestérifiés par le butanol,
d) les terpolymères acide acrylique/acrylate d'éthyle/N-tert-butylacrylamide, et
e) les copolymères acétate de vinyle/acide crotonique, les terpolymères acétate de vinyle/acide crotonique/néodécanoate de vinyle, le tert-butyl-4-benzoate de vinyle et leurs mélanges.

Bien entendu, le choix du ou des type(s) de polymère(s) filmogène(s) utilisé(s) dépendra du type de milieu physiologiquement acceptable choisi pour la composition.

Dans la composition, la teneur en polymère(s) filmogène(s) peut varier de 0,1% à 15 % en poids, notamment de 5 % à 10 % en poids, et en particulier être inférieure ou égale à 7 % en poids par rapport au poids total de la composition.

### Agent auxiliaire de filmification

L'agent auxiliaire de filmification peut être choisi parmi tous les composés connus de l'homme du métier comme étant susceptibles de remplir la fonction recherchée, et être notamment choisi parmi les agents plastifiants et les agents de coalescence du polymère filmogène.

En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- les esters d'acides notamment carboxyliques tels que les citrates, notamment le citrate de triéthyle, le citrate de tributyle, l'acétylcitrate de triéthyle, l'acétylcitrate de tributyle, l'acétylcitrate de triéthyl-2 hexyle ; les phtalates, notamment le phtalate de diéthyle, le phtalate de dibutyle, le phtalate de dioctyle, le phtalate de dipentyle, le phtalate de diméthoxyéthyle ; les phosphates, notamment le phosphate de tricrésyle, le phosphate de tributyle, le phosphate de triphényle, le phosphate de tributoxyéthyle ; les tartrates, notamment le tartrate de dibutyle ; les adipates ; les carbonates ; les sébaçates ; le benzoate de benzyle, l'acétylricinoléate de butyle, l'acétylricinoléate de glycéryle, le glycolate de butyle, le camphre, le triacétate de glycérol, le N-éthyl-o,p-toluènesulfonamide,
- les dérivés oxyéthylénés tels que les huiles oxyéthylénées, notamment les huiles végétales telles que l'huile de ricin; les huiles de silicone,
- leurs mélanges.

Le type et la quantité d'agent plastifiant et/ou de coalescence peut être choisie par l'homme du métier sur la base de ses connaissances générales, de manière à obtenir une composition ayant des propriétés cosmétiquement acceptables, sous réserve bien entendu que la teneur totale en agent texturant ne dépasse pas celle indiquée précédemment et que ladite composition conserve sa caractéristique de fluidité et/ou d'aptitude à former un film ayant une faible résistance à l'usure.

Par exemple, la teneur en agent plastifiant et/ou de coalescence peut aller de 0,01 % à 10 % et en particulier de 1 % à 3 % en poids par rapport au poids total de la composition.

### Agent épaississant

L'agent épaississant peut en particulier être un épaississant de phase non aqueuse et peut être choisi parmi : les silices hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610 ®", "CAB-O-SIL TS-720® "par la société Cabot, "AEROSIL R972®", "AEROSIL R974®" par la société Degussa ; les argiles telles que la montmorillonite, les argiles modifiées telles que les bentones par exemple, l'hectorite stéaralkonium, la bentonite stéaralkonium , les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

L'agent épaississant peut être également un épaississant de phase aqueuse tel que par exemple une argile et/ou un polymère gélifiant aqueux.

Le choix de ces agents épaississants est bien entendu réalisé en tenant compte de la nature du milieu physiologiquement acceptable.

La proportion totale en agent(s) épaississant(s) dans les compositions selon l'invention est généralement inférieure ou égale à 5 %, notamment à 2 %, et en particulier à 1 % en poids par rapport au poids total de la composition.

Le choix des agents texturants utilisés dans la composition selon l'invention est bien entendu réalisé en tenant compte de la nature du milieu physiologiquement acceptable de la composition.

### Les particules à reflet métallique

Par « particules à reflet métallique », on désigne des particules dont la nature, la taille, la structure et l'état de surface leur permettent de réfléchir la lumière incidente notamment de façon non iridescente.

Les particules à reflet métallique utilisables dans les compositions selon l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

Parmi les dérivés métalliques pouvant être présents dans lesdites particules, on peut citer notamment les oxydes métalliques tels que par exemple les oxydes de titane, notamment TiO₂, de fer notamment Fe₂O₃, d'étain, de chrome et les composés suivants : MgF₂, TiCl₄, CrF₃, ZnS, ZnSe, Al₂O₃, MgO, SeO₃, ZrO₂, MoS₂ et leurs mélanges ou alliages.

Selon une première variante, les particules à reflet métallique sont composées d'au moins un métal tel que défini précédemment, d'au moins un dérivé métallique tel que défini précédemment ou bien encore de leur mélange.

Ces particules peuvent être au moins partiellement recouvertes par une couche d'un autre matériau, par exemple de matériau transparent tel que notamment du colophane, de la silice, des stéarates, des polysiloxanes, des résines polyesters, des résines époxydiques, des résines polyuréthanes et des résines acryliques.

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER® de la société ECKART et les particules d'alliage métallique comme des poudres de bronze (alliage cuivre et zinc) enrobé de silice commercialisées sous la dénomination de Visionaire Bright Natural Gold® de la société Eckart.

Selon une seconde variante, les particules à reflet métallique sont des particules qui comportent un substrat et qui présentent donc une structure multicouche par exemple bicouche. Ce substrat peut être organique ou minéral, naturel ou synthétique, monomatière ou multimatériaux, plein ou creux. Lorsque le substrat est synthétique, il peut être réalisé avec une forme favorisant la formation d'une surface réfléchissante après revêtement, notamment après le dépôt d'une couche de matériaux à reflet métallique. Le substrat peut, par exemple, présenter une surface plane et la couche de matériaux à reflet métallique une épaisseur sensiblement uniforme.

Le substrat peut être en particulier choisi parmi les métaux et les dérivés métalliques tels que cités précédemment, et également parmi les verres, les céramiques, les alumines, les silices, les silicates et notamment aluminosilicates et les borosilicates, le mica synthétique tel que le fluorophlogopite, et leurs mélanges, cette liste n'étant pas limitative.

La couche à reflet métallique peut enrober en totalité ou en partie le substrat et cette couche peut être au moins partiellement recouverte par une couche d'un autre matériau, par exemple un matériau transparent notamment tel que cité précédemment. Selon un mode de réalisation particulier, la couche à reflet métallique enrobe en totalité, directement ou indirectement, c'est-à-dire avec interposition d'au moins une couche intermédiaire métallique ou non, le substrat.

Les métaux ou dérivés métalliques pouvant être utilisés dans la couche à reflet métallique sont tels que définis précédemment.

Des particules de verre recouvertes d'une couche métallique sont décrites notamment dans les documents JP-A-09188830, JP-A-10158450, JP-A-10158541, JP-A-07258460 et JP-A-05017710.

A titre illustratif de ces particules comportant un substrat de verre, on peut citer celles revêtues respectivement d'argent, d'or ou de titane, en forme de plaquettes, commercialisées par la société NIPPON SHEET GLASS sous les dénominations MICROGLASS METASHINE® , celles revêtues soit d'oxyde de fer brun, soit d'oxyde de titane, d'oxyde d'étain ou d'un de leur mélange comme celles commercialisées sous la dénomination REFLECKS® par la société ENGELHARD ou celles commercialisées sous la référence METASHINE MC 2080GP® par la société NIPPON SHEET GLASS.

Ces particules de verre recouvertes de métaux peuvent être enrobées de silice comme celles commercialisées sous la dénomination METASHINE® série PSS1 ou GPS1 par la société NIPPON SHEET GLASS.

Parmi les particules à reflet métallique comportant un substrat, utilisables dans les compositions selon l'invention, on peut encore citer les particules à multicouches interférentielles.

Des exemples de structure multicouche comprenant une couche métallique utilisables dans des compositions réalisées conformément à l'invention sont par exemple les structures suivantes : Al/SiO₂/Al/SiO₂/Al, des particules ayant cette structure étant commercialisées par la société DUPONT DE NEMOURS ; Cr/MgF₂/Al/MgF₂/Cr, des particules ayant cette structure étant commercialisées sous la dénomination de CHROMAFLAIR® par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des particules ayant cette structure étant commercialisées sous la dénomination de SICOPEARL® par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des particules ayant ces structures étant commercialisées sous la dénomination XIRONA® par la société MERCK (DARMSTADT).

Selon un mode de réalisation particulier, la composition conforme à l'invention est exempte de particules de dioxyde de silicium enrobées d'oxyde métallique et plus généralement de métal ou de dérivé métallique.

Les particules à reflet métallique utilisées dans les compositions selon l'invention peuvent présenter des formes variées. Elles peuvent être en particulier planes. Par « particules planes », on désigne des particules dont la surface ne présente sensiblement ni aspérité ni inégalité et qui ne présentent qu'une faible courbure voire une courbure nulle.

Ces particules peuvent en particulier se présenter sous forme de plaquettes. Par « plaquettes », on désigne des particules dont le rapport de la plus grande dimension à la plus petite dimension, appelé facteur de forme, est supérieur ou égal à 5.

Par « dimensions », on désigne les dimensions données par la distribution granulométrique statistique à la moitié de la population, dite D50.

Les particules à reflet métallique ont en particulier un facteur de forme supérieur ou égal à 8 et notamment à 10, et par exemple supérieur ou égal à 15.

Les particules à reflet métallique utilisées dans les compositions selon l'invention ont par exemple selon leur plus grande dimension, une taille moyenne inférieure ou égale à 25 µm, en particulier inférieure ou égale à 10 µm et notamment d'environ 6 µm.

Par « taille moyenne », on désigne la dimension donnée par la distribution granulométrique statistique à la moitié de la population, dite D50.

Lesdites particules ont généralement une épaisseur inférieure ou égale à 1 µm, notamment inférieure ou égale à 0,7 µm, en particulier inférieure ou égale à 0,5 µm.

La proportion totale en particules à reflet métallique est généralement supérieure ou égale à 2 %, notamment supérieure ou égale à 3 %, en particulier supérieure ou égale à 5 % et plus particulièrement supérieure ou égale à 7 % en poids par rapport au poids total de la composition. La proportion totale en particules à reflet métallique est notamment inférieure ou égale à 70 %, en particulier inférieure ou égale à 20 % en poids et plus particulièrement inférieure ou égale à 10 % en poids par rapport au poids total de la composition.

### Milieu physiologiquement acceptable

La composition selon l'invention comprend en outre un milieu physiologiquement acceptable. On désigne ainsi un milieu non toxique et notamment susceptible d'être appliqué sur les phanères d'êtres humains.

Ce milieu peut être de type solvant organique, aqueux ou mixte.

Le milieu physiologiquement acceptable de la composition comprend généralement au moins un solvant volatil. Le solvant volatil peut être notamment choisi parmi les solvants organiques volatils, l'eau et leurs mélanges

### Solvants organiques

La composition selon l'invention peut comprendre au moins un milieu solvant organique constituant une phase organique, composé d'au moins un solvant organique volatil à température ambiante.

Comme solvant organique volatil ou non à température ambiante, on peut citer :
- les cétones liquides à température ambiante tels que la méthyléthylcétone, la méthylisobutylcétone, la diisobutylcétone, l'isophorone, la cyclohéxanone, et l'acétone ;
- les alcools liquides à température ambiante tels que l'éthanol, l'isopropanol, le butanol, le diacétone alcool, le 2-butoxyéthanol, et le cyclohexanol ;
- les glycols liquides à température ambiante tels que l'éthylène glycol, le propylène glycol, le pentylène glycol, et le glycérol ;
- les éthers de propylène glycol liquides à température ambiante tels que le monométhyléther de propylène glycol, l'acétate de monométhyl éther de propylène glycol, et le mono n-butyl éther de dipropylène glycol ;
- les esters à chaînes courtes (ayant de 3 à 8 atomes de carbone au total) tels que l'acétate de méthyle, l'acétate d'éthyle, l'acétate de propyle, l'acétate de n-butyle, l'acétate d'isopentyle et l'acétate d'aryle;
- les alcanes liquides à température ambiante tels que le décane, l'heptane, l'octane, le dodécane, le cyclohexane, et l'isododécane ;
- les aldéhydes liquides à température ambiante, tels que le benzaldéhyde et l'acétaldéhyde, et
- leurs mélanges.

Le solvant est en particulier choisi parmi les esters à chaîne courte (ayant de 3 à 8 atomes de carbone au total), les alcools liquides à température ambiante, et leurs mélanges.

La composition selon l'invention peut également contenir une ou plusieurs huiles de silicones, généralement en faible quantité, c'est-à-dire pouvant être inférieure à 10 % en poids de la phase solvant. Il peut notamment s'agir d'huiles volatiles ou non telles que les diméthicones, phényldiméthicones, alkyldiméthicones, diméthicone copolyols ou cyclométhicones.

Lorsque le milieu physiologiquement acceptable comprend une quantité significative en phase organique, celle-ci peut être présente en une proportion variant de 30 à 97 % en poids et notamment allant de 50 à 95 % en poids par rapport au poids total de la composition.

### Phase aqueuse

La composition selon l'invention peut aussi comprendre au moins un milieu aqueux, constituant une phase aqueuse, qui peut former la phase continue de la composition.

La phase aqueuse peut être constituée essentiellement d'eau ; elle peut également comprendre un mélange d'eau et de solvant(s) organique(s) miscible(s) à l'eau (miscibilité dans l'eau supérieure à 50 % en poids à 25 °C) comme les monoalcools inférieure ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.

La phase aqueuse (eau et éventuellement solvant organique miscible à l'eau) peut être présente en une proportion variant de 30 à 97 % en poids, notamment allant de 50 à 80 % en poids par rapport au poids total de la composition.

### Additifs

Les compositions selon l'invention peuvent comprendre en outre au moins un additif notamment une matière colorante additionnelle choisie parmi les nacres naturelles ou synthétiques, les pigments non métalliques, les particules non métalliques (ayant comme support par exemple : verre, polyacrylate, polyuréthane, polybutylène téréphtalate), les fibres naturelles ou synthétiques et les colorants hydrosolubles ou liposolubles.

La composition pour le maquillage des ongles selon l'invention est plus particulièrement une composition de vernis à ongles. Cette composition peut être appliquée sur des ongles naturels ou synthétiques tels que des faux ongles.

La présente invention a en outre pour objet l'utilisation d'une composition telle que définie précédemment pour former une couche de base, encore appelée « base-coat » en terminologie anglo-saxonne, dans un maquillage multicouche.

### Kit de maquillage

La présente invention a encore pour objet un kit pour le maquillage des ongles, qui selon une première variante, comprend, dans des conditionnements séparés, une première composition comprenant, dans un premier milieu physiologiquement acceptable, des particules à reflet métallique en une proportion supérieure ou égale à 2 % en poids, et notamment supérieure à 3 % en poids par rapport au poids total de la première composition et au moins un agent texturant ou un mélange d'agents texturants en une proportion inférieure ou égale à 15 % en poids par rapport au poids total de la composition, et une seconde composition, différente de la première composition, comprenant, dans un second milieu physiologiquement acceptable, au moins 10 % en poids par rapport au poids total de ladite seconde composition d'au moins un agent filmogène.

Selon une seconde variante, l'invention a pour objet un kit pour le maquillage des ongles qui comprend dans des conditionnements séparés, une première composition comprenant dans un premier milieu physiologiquement acceptable, des particules à reflet métallique, ladite première composition étant apte à former un film dont la résistance à l'usure, mesurée selon norme AFNOR NF T30-015, est supérieure à 5 % en poids, en particulier à 10 % en poids et plus particulièrement supérieure à 15 % en poids, et une seconde composition, différente de la première composition, comprenant dans un milieu physiologiquement acceptable, au moins 10 % en poids par rapport au poids de la seconde composition d'au moins un agent filmogène.

Quelle que soit la variante du kit envisagée, la première composition peut être une composition pour le maquillage des ongles telle que définie précédemment.

La seconde composition du kit peut, selon un mode de réalisation particulier, comprendre au moins 15 % en poids par rapport au poids total de la seconde composition d'agent(s) filmogène(s).

Il s'agit d'agents filmogènes usuels et plus particulièrement des polymères filmogènes solubles ou dispersibles dans les solvants organiques ou de latex filmogène, notamment tels que décrits précédemment. Le choix de ces agents filmogènes est également réalisé en fonction de la nature du milieu physiologiquement acceptable de ces secondes compositions. Les secondes compositions du kit de l'invention peuvent également comprendre au moins un agent auxiliaire de filmification, tels que ceux décrits précédemment.

L'agent plastifiant et/ou de coalescence peut être présent dans la composition en une teneur allant de 0,01 à 15 % en poids, en particulier de 2 à 7 % en poids par rapport au poids total de la composition

La seconde composition peut comprendre en outre au moins un agent épaississant usuel, notamment choisi parmi ceux décrits précédemment.

La proportion en agent(s) épaississant(s) présent(s) dans la seconde composition peut aller de 0,01 à 3 % en poids par rapport au poids total de la compostion.

Le milieu physiologiquement acceptable de la seconde composition de l'invention est, comme pour celui de la première composition, un milieu non toxique notamment susceptible d'être appliqué sur les phanères d'êtres humains. Ce milieu peut être de type organique, aqueux ou mixte. Il comprend généralement au moins un solvant volatil qui peut être notamment choisi parmi les solvants organiques volatils, l'eau, et leurs mélanges.

A titre d'exemple de solvants utilisables dans ladite seconde composition, on peut citer ceux décrits précédemment.

Toutefois, bien entendu, l'homme du métier veillera à choisir ce second milieu physiologiquement acceptable, de telle sorte que l'application de la seconde composition sur le film susceptible d'être généré par l'application de la première composition puis séchage, n'affecte pas ledit film et en particulier ne le dissout pas ou ne le dilue pas.

La phase aqueuse (eau et éventuellement le solvant organique miscible à l'eau), la phase organique ou la somme de ces deux phases, peut être présente en une teneur totale comprise entre 1 et 90 % en poids, notamment allant de 5 à 60 % en poids, et en particulier allant de 15 à 40 % en poids, par rapport au poids total de ladite seconde composition.

La seconde composition ne doit pas faire obstacle à la manifestation de l'effet miroir recherché.

Généralement, la seconde composition du kit selon l'invention est translucide, semi-transparente ou transparente.

Elles peuvent encore être qualifiées de translucide ou transparente dans la masse. Cette propriété de transparence ou de translucidité dans la masse signifie qu'une couche d'une épaisseur fixée arbitrairement à 1 cm, laisse passer une partie de la lumière visible soit en la diffusant (compositions translucides dans la masse), soit sans la diffuser (compositions transparentes dans la masse). Dans ce cas on mesure la densité optique en utilisant des cuves en quartz de 10 mm d'épaisseur. La valeur de la densité optique doit être inférieure à 1.

La seconde composition peut également comprendre une matière colorante qui peut être choisie parmi les colorants solubles dans ledit second milieu physiologiquement acceptable, et éventuellement les composés pulvérulents.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, et le jaune quinoléine.

Les composés pulvérulents peuvent être choisis parmi les pigments et/ou les nacres naturelles ou synthétiques et/ou les paillettes et/ou les fibres, habituellement utilisés dans les compositions cosmétiques pour le maquillage des ongles, telles que les vernis à ongles.

Les pigments peuvent être blancs ou colorés, minéraux et/ou organiques. Parmi ces pigments, on peut citer le noir de carbone, les pigments de type D & C, et les laques à base de carmin de cochenille, de baryum, strontium, calcium, aluminium, et la guanine.

Par « nacres », on désigne des particules irisées, en particulier produites par certains mollusques dans leur coquille ou bien synthétisées.

Les paillettes et/ou les fibres peuvent être choisies parmi celles en matériaux de type résine acrylique, polyester, polyéthylène téréphtalate, ou polyuréthane.

La matière colorante peut être présente en une teneur allant de 0,01 % à 10 % en poids, par rapport au poids total de la composition.

Les proportions sont données à titre général, mais il est bien entendu que l'homme du métier veillera à ce que la nature de ces composés et leur concentration n'affectent pas substantiellement l'effet miroir de la première composition.

La seconde composition du kit selon l'invention peut comprendre en outre au moins un additif choisi parmi, les agents d'étalements, les agents mouillants, les agents dispersants, les anti-mousses, les conservateurs, les filtres UV, les actifs, les tensioactifs, les parfums, les neutralisants, les stabilisants et les antioxydants.

Cette seconde composition est plus particulièrement destinée à être utilisée comme composition de finition pour le maquillage, encore appelée « top-coat » en terminologie anglo-saxonne, et en particulier comme composition de finition de vernis à ongles. Toutefois, elle peut également être mise en oeuvre à titre de couche de base. Dans ce cas, il lui est superposé au moins une couche de la première composition.

Les ingrédients de ladite seconde composition, et leur concentration, peuvent être choisis par l'homme du métier, sous réserve des conditions mentionnées précédemment pour l'obtention de l'effet miroir recherché. En particulier, ces ingrédients seront choisis pour que ladite seconde composition forme, après application, un film brillant, adhérent et présentant une résistance à l'usure satisfaisante.

Les compositions de la présente invention peuvent être obtenues selon les procédés de préparation classiquement utilisés en cosmétique.

La présente invention a encore pour objet un procédé pour le maquillage des ongles comprenant l'application sur tout ou partie de la surface à maquiller d'au moins une couche de la première composition du kit défini précédemment ou d'une composition cosmétique pour le maquillage des ongles telle que définie précédemment.

La présente invention a également pour objet un procédé pour le maquillage des ongles comprenant l'application d'au moins une couche de chacune des deux compositions d'un kit tel que défini précédemment.

Généralement, il est appliqué au moins une couche, voire deux couches, de la première composition, à laquelle est ensuite superposée au moins une couche, voire deux couches, de la seconde composition. Toutefois, il est également possible de procéder à une inversion de l'ordre de superposition de ces compositions, c'est-à-dire de privilégier d'abord le dépôt d'une ou deux couches de la seconde composition à titre de couche de base et l'application consécutive d'une ou deux couches de la première composition. Toutefois, dans ce cas particulier, il est souhaitable de procéder à une nouvelle application d'une couche ou de deux couches de la seconde composition à la surface de la couche métallique. Ce mode d'application, impliquant la superposition de plusieurs couches des deux compositions, est particulièrement avantageux pour améliorer le lissage de la couche métallique, et en particulier les propriétés du maquillage ainsi obtenu en terme de brillance et/ou de tenue.

La première composition peut être appliquée de manière uniforme ou non uniforme, c'est-à-dire de façon discontinue notamment sous forme de motifs géométriques, symétriques ou dissymétriques (par exemple sous forme de points, carrés, ronds, étoiles), répartis de façon aléatoire ou ordonnée à contours précis ou flous sur la surface à maquiller de l'ongle.

Après application de la première composition, il est préférable de laisser un temps suffisant jusqu'à obtention d'un film solide avant d'appliquer la seconde composition.

La seconde composition est généralement appliquée de manière uniforme sur la surface à maquiller, même s'il est toutefois possible de ne l'appliquer que sur le film formé par la première composition.

Dans le procédé de l'invention, ledit support à maquiller est un ongle naturel ou synthétique.

La présente invention a encore pour objet un support maquillé comprenant un maquillage susceptible d'être obtenu par le procédé défini précédemment. Le support est en particulier un accessoire de maquillage de l'ongle, notamment un faux-ongle.

Les exemples donnés ci-après sont donnés à titre illustratif et sans caractère limitatif.

### Exemple 1 : Exemple de réalisation d'un vernis à ongles

On prépare selon les méthodes conventionnelles les compositions suivantes :

### a) Composition pour former une couche de base à effet miroir

- Dispersion de particules d'aluminium commercialisée sous la dénomination de STARBRITE 1200 EAC® par la société SIBERLINE* 40 g
- acétobutyrate de cellulose (agent filmogène) 4 g
- bentone (agent rhéologique) 0,5 g
- acétate d'éthyle (solvant) qsp 100 g

* STARBRITE 1200 EAC® est un produit contenant 20 % en poids de matière sèche dans 80 % d'acétate d'éthyle.

### b) Composition de finition

- Acétobutyrate de cellulose (agent filmogène) 15 g
- Ethyl tosylamide (agent plastifiant) 3 g
- Diméthicone 0,2 g
- Alcool éthylique qsp 100 g

La composition pour former la couche de base à effet miroir est appliquée en continu sur des ongles démaquillés, sous forme de monocouche. Après séchage, on obtient un film de maquillage présentant un effet miroir marqué.

Puis la seconde composition est appliquée sur le film formé par la couche de base. Après séchage, on obtient un maquillage à effet miroir marqué, très brillant présentant une résistance à l'usure satisfaisante.

## Revendications

1. Composition pour le maquillage des ongles, **caractérisée par le fait qu'**elle comprend, dans un milieu physiologiquement acceptable, des particules à reflet métallique en une proportion supérieure ou égale à 2 % en poids, par rapport au poids total de la composition, et au moins un agent texturant ou un mélange d'agents texturants en une proportion inférieure ou égale à 15 % en poids, par rapport au poids total de la composition.

2. Composition pour le maquillage des ongles, **caractérisée par le fait qu'**elle comprend, dans un milieu physiologiquement acceptable, des particules à reflet métallique et au moins un agent texturant ou un mélange d'agents texturants en une proportion inférieure ou égale à 15 % en poids par rapport au poids total de la composition, et que ladite composition est apte à former un film dont la résistance à l'usure exprimée en perte de masse PM mesurée selon la norme AFNOR NF T30-015, est supérieure à 5 % en poids, en particulier supérieure à 10 % en poids et plus particulièrement supérieure à 15 % en poids.

3. Composition selon la revendication 2, **caractérisée par le fait que** lesdites particules à reflet métallique sont présentes en une proportion supérieure ou égale à 2 % en poids par rapport au poids total de la composition.

4. Composition selon l'une quelconque des revendications précédentes, **caractérisée par le fait que** la proportion totale en particules à reflet métallique est supérieure ou égale à 5 %, et en particulier à 7 % en poids par rapport au poids total de la composition.

5. Composition selon l'une quelconque des revendications précédentes, **caractérisée par le fait que** la proportion totale en particules à reflet métallique est inférieure ou égale à 70 %, notamment à 20 % en poids, et en particulier à 10 % en poids par rapport au poids total de la composition.

6. Composition selon l'une quelconque des revendications précédentes, **caractérisée par le fait que** lesdites particules à reflet métallique sont choisies parmi :
- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

7. Composition selon la revendication 6, **caractérisée par le fait que** ledit métal est choisi parmi : Ag, Au, Cu, Al, Zn, Ni, Mo, Cr et leurs mélanges ou alliages.

8. Composition selon la revendication 6, **caractérisée par le fait que** ledit dérivé métallique est choisi parmi les oxydes de titane, notamment TiO₂, de fer, notamment Fe₂O₃, des oxydes d'étain, de chrome et les composés MgF₂, TiCl₄, CrF₃, ZnS, ZnSe, Al₂O₃, MgO, SeO₃, ZrO₂ et MoS₂ et leurs mélanges ou alliages.

9. Composition selon la revendication 6, **caractérisée par le fait que** ledit substrat est choisi parmi les métaux, les dérivés métalliques, les verres, les céramiques, les alumines, les silices, les silicates, notamment les aluminosilicates et les borosilicates, le mica synthétique tel que le fluorophlogotipe, et leurs mélanges.

10. Composition selon l'une quelconque des revendications 6 à 9, **caractérisée par le fait que** lesdites particules comportant un substrat ont une structure bicouche ou multicouche.

11. Composition selon l'une quelconque des revendications précédentes, **caractérisée par le fait que** lesdites particules à reflet métallique sont planes.

12. Composition selon l'une quelconque des revendications précédentes, **caractérisée par le fait que** lesdites particules à reflet métallique se présentent sous forme de plaquettes.

13. Composition selon l'une quelconque des revendications précédentes, **caractérisée par le fait que** lesdites particules à reflet métallique ont un facteur de forme supérieur ou égal à 8, notamment à 10 et par exemple supérieur ou égal à 15.

14. Composition selon l'une quelconque des revendications précédentes, **caractérisée par le fait que** lesdites particules à reflet métallique ont, selon leur plus grande dimension, une taille moyenne inférieure ou égale à 25 µm, en particulier inférieure ou égale à 10 µm et notamment d'environ 6 µm.

15. Composition selon l'une quelconque des revendications précédentes, **caractérisée par le fait que** la proportion totale en agents texturants est inférieure ou égale à 10 % en poids par rapport au poids total de la composition.

16. Composition selon l'une quelconque des revendications précédentes, **caractérisée par le fait que** la proportion totale en agents texturants est supérieure ou égale à 2 %, en particulier à 5 % en poids par rapport au poids total de la composition.

17. Composition selon l'une quelconque des revendications précédentes, **caractérisée par le fait que** lesdits agents texturants sont choisis parmi les agents filmogènes, les résines, les agents auxiliaires de filmification, les agents épaississants et leurs mélanges.

18. Composition selon la revendication 17, **caractérisée par le fait que** ledit agent filmogène comprend au moins un polymère filmogène.

19. Composition selon la revendication 18, **caractérisée par le fait que** ledit polymère filmogène est choisi dans le groupe constitué par les polymères radicalaires, les polycondensats et les polymères d'origine naturelle.

20. Composition selon l'une quelconque des revendications 18 et 19, **caractérisée par le fait que** la proportion totale en polymère(s) filmogène(s) peut varier de 0,1 à 15 % en poids, notamment de 5 à 10 % en poids, et en particulier être inférieure ou égale à 7 % en poids par rapport au poids total de la composition.

21. Composition selon la revendication 17, **caractérisée par le fait que** ledit agent épaississant est choisi dans le groupe constitué par les épaississants de phase non aqueuse tels que les silices hydrophobes, les argiles éventuellement modifiées et les alkyléthers de polysaccharides, et les épaississants de phase aqueuse tels que les argiles et les polymères gélifiants aqueux.

22. Composition selon l'une quelconque des revendications 17 et 21, **caractérisée par le fait que** la proportion totale en agent(s) épaississant(s) est inférieure ou égale à 5 %, notamment à 2 %, et en particulier à 1 % en poids par rapport au poids total de la composition.

23. Composition selon l'une quelconque des revendications précédentes, **caractérisée par le fait qu'**elle comprend au moins un milieu solvant organique et/ou au moins un milieu aqueux.

24. Composition selon la revendication 23, **caractérisée par le fait que** ledit milieu comprend au moins un solvant volatil.

25. Composition selon l'une quelconque des revendications précédentes, **caractérisée par le fait que** ladite composition comprend en outre au moins un additif, notamment une matière colorante additionnelle choisie parmi les nacres naturelles ou synthétiques, les pigments non métalliques, les particules non métalliques, les fibres naturelles ou synthétiques et les colorants hydrosolubles ou liposolubles.

26. Utilisation d'une composition telle que définie selon l'une quelconque des revendications précédentes pour former une couche de base dans un maquillage multicouche.

27. Kit pour le maquillage des ongles, **caractérisé par le fait qu'**il comprend, dans des conditionnements séparés, une première composition comprenant, dans un premier milieu physiologiquement acceptable, des particules à reflet métallique en une proportion supérieure ou égale à 2 % en poids par rapport au poids total de la première composition et au moins un agent texturant ou un mélange d'agents texturants en une proportion inférieure ou égale à 15 % en poids par rapport au poids total de la composition, et une seconde composition, différente de la première composition, comprenant, dans un second milieu physiologiquement acceptable, au moins 10 % en poids par rapport au poids total de la seconde composition d'au moins un agent filmogène.

28. Kit selon la revendication 27, **caractérisé par le fait que** ladite première composition est telle que définie dans l'une quelconque des revendications 3 à 25.

29. Kit pour le maquillage des ongles, **caractérisé par le fait qu'**il comprend, dans des conditionnements séparés, une première composition comprenant, dans un premier milieu physiologiquement acceptable, des particules à reflet métallique, ladite première composition étant apte à former un film dont la résistance à l'usure exprimée en perte de masse, mesurée selon la norme AFNOR NF T30-015, est supérieure à 5 % en poids, en particulier à 10 % et plus particulièrement supérieure à 15 % en poids, et une seconde composition, différente de la première composition, comprenant dans un second milieu physiologiquement acceptable, au moins 10 % en poids par rapport au poids de la seconde composition d'au moins un agent filmogène.

30. Kit selon la revendication 29, **caractérisé par le fait que** ladite première composition est telle que définie dans l'une quelconque des revendications 2 à 25.

31. Kit selon l'une quelconque des revendications 27 à 30, **caractérisé par le fait que** ladite seconde composition comprend au moins 15 % en poids par rapport au poids total de la seconde composition d'agent(s) filmogène(s).

32. Kit selon l'une quelconque des revendications 27 à 31, **caractérisé par le fait que** ladite seconde composition comprend en outre au moins un agent épaississant.

33. Kit selon l'une quelconque des revendications 27 à 32, **caractérisé par le fait que** ledit second milieu physiologiquement acceptable comprend au moins un milieu solvant organique et/ou au moins un milieu aqueux.

34. Kit selon l'une quelconque des revendications 27 à 33, **caractérisé par le fait que** ladite seconde composition est translucide, semi-transparente ou transparente.

35. Kit selon l'une quelconque des revendications 27 à 34, **caractérisé par le fait que** ladite seconde composition comprend au moins une matière colorante choisie parmi les colorants solubles dans ledit second milieu physiologiquement acceptable et les composés pulvérulents tels qu'au moins un pigment et/ou au moins une nacre et/ou au moins des paillettes et/ou au moins des fibres.

36. Kit selon l'une quelconque des revendications 27 à 35, **caractérisé par le fait que** ladite seconde composition comprend en outre au moins un additif choisi dans le groupe constitué par les agents d'étalement, les agents mouillants, les agents dispersants, les anti-mousses, les conservateurs, les filtres UV, les actifs, les tensioactifs, les parfums, les neutralisants, les stabilisants et les anti-oxydants.

37. Procédé pour le maquillage des ongles **caractérisé par le fait qu'**il comprend l'application sur tout ou partie de la surface à maquiller d'au moins une couche d'une composition telle que définie dans l'une quelconque des revendications 1 à 25.

38. Procédé pour le maquillage des ongles **caractérisé par le fait qu'**il comprend l'application sur la surface à maquiller d'au moins une couche de chacune des compositions d'un kit tel que défini dans l'une quelconque des revendications 27 à 36.

39. Procédé de maquillage selon la revendication 37 ou 38, **caractérisé par le fait que** la surface à maquiller est un ongle naturel ou synthétique.

40. Support synthétique maquillé comprenant un maquillage susceptible d'être obtenu selon le procédé tel que défini selon l'une quelconque des revendications 37 à 39.

41. Support synthétique maquillé selon la revendication 40, **caractérisé par le fait qu'**il s'agit de faux ongles.
